(19) Europäisches Patentamt
European Patent Office
Office européen des brevets

(11) EP 1 302 199 A2

(12) DEMANDE DE BREVET EUROPEEN

(43) Date de publication:
**16.04.2003 Bulletin 2003/16**

(51) Int Cl.$^7$: **A61K 7/48**, A61K 7/42

(21) Numéro de dépôt: **02292395.7**

(22) Date de dépôt: **27.09.2002**

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **16.10.2001 FR 0113335**
**16.10.2001 FR 0113334**
**16.10.2001 FR 0113336**

(71) Demandeur: **L'OREAL**
**75008 Paris (FR)**

(72) Inventeurs:
- **Forestier, Serge**
**77410 Claye Souilly (FR)**
- **Candau, Didier**
**91570 Bievres (FR)**
- **Seyler, Nathalie**
**94700 Maisons-Alfort (FR)**
- **Elguidj, Irène**
**92200 Neuilly (FR)**

(74) Mandataire: **Miszputen, Laurent**
**L'OREAL-DPI**
**6 rue Bertrand Sincholle**
**92585 Clichy Cédex (FR)**

# (54) Compositions pour la coloration de la peau proche du bronzage naturel a base d'un pigment du type monascus et utilisations

(57) La présente invention se rapporte à des compositions cosmétiques et/ou dermatologiques produisant sur la peau une coloration durable et non-couvrante, proche du bronzage naturel, caractérisée par le fait qu'elle comprend, dans un support cosmétiquement acceptable, au moins un pigment susceptible d'être obtenu par extraction par un solvant organique ou hydro-organique d'un milieu de culture de micromycètes du type monascus.

L'invention concerne également les applications de ces compositions à la coloration durable et non-couvrante de la peau proche du bronzage naturel de la peau.

EP 1 302 199 A2

## Description

La présente invention se rapporte à des compositions cosmétiques et/ou dermatologiques produisant sur la peau une coloration durable et non-couvrante, proche du bronzage naturel, caractérisée par le fait qu'elle comprend, dans un support cosmétiquement acceptable, au moins un pigment susceptible d'être obtenu par extraction par un solvant organique ou hydro-organique d'un milieu de culture de micromycètes du type monascus.

L'invention concerne également les applications de ces compositions à la coloration de la peau proche du bronzage naturel de la peau.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés.

A cet effet, on sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. Il existe donc une demande croissante de produits autobronzants agissant rapidement et conférant une coloration plus proche du bronzage naturel.

Ainsi, on est toujours à la recherche de nouveaux composés et de nouvelles compositions permettant de conférer artificiellement à la peau une coloration proche du bronzage naturel d'une manière simple, efficace, rapide et sans risque.

Les pigments issus de champignons filamenteux du type monascus sont connus depuis longtemps comme agents colorants de produits alimentaires, de produits pharmaceutiques et cosmétiques. Les champignons filamenteux du type monascus sont obtenus par fermentation. Les souches habituellement utilisées sont les souches du genre *Monascus anka, Monascus purpurea, Monascus ruber, Monascus pilosus.* Ces micromycètes pigmentogènes produisent six sortes de pigments qui diffèrent par leur degré d'insaturation , leurs fonctions auxochromes et la taille de leur chaîne aliphatique. Ces pigments peuvent être intracellulaires ou extracellullaires et possèdent des teintes différentes suivant leur structure chimique. On distingue les pigments jaunes du type ankaflavine et du type monascine ; les pigments oranges du type monascorubrine et rubropunctatine et les pigments rouges du type monascorubramine et rubropunctamine. Les pigments oranges proviennent directement du métabolisme secondaire du champignon tandis que les pigments jaunes et les pigments rouges sont des métabolites issus d'une réaction de réduction des pigments oranges pour l'obtention des pigments jaunes et d'une réaction d'amination pour les pigments rouges.

On connaît dans la demande de brevet JP 4-23880 des compositions cosmétiques contenant un extrait issu de la souche mutante R300-30 du Monascus anka (FERM n° 11135) et éventuellement d'autres filtres UV classiques; ledit extrait étant utilisé pour ses propriétés d'absorption des UVA et des UVB. Ce document ne mentionne pas l'application de ces compositions pour la coloration artificielle de la peau.

On connaît dans la demande de brevet JP 64-90109 des compositions cosmétiques contenant un extrait liquide de culture d'une moisissure du genre Monascus. Cet extrait liquide contient des enzymes protéolytiques, des vitamines, des peptides et divers acides aminés. Ces compositions sont utilisées pour leurs propriétés hydratantes et détergentes sur la peau.

On connaît dans les demandes de brevet JP 60-149512 et JP10-194928 des compositions de maquillage contenant des pigments de Monascus liés à une poudre de fibroïne de soie régénérée. Ce document ne mentionne pas l'application de ces compositions pour la coloration artificielle de la peau.

On connaît dans la demande de brevet JP 57-145159 des compositions cosmétiques de pour le soin du visage à base de pigments naturels (par exemple Monascus) liés à une poudre de silicate d'aluminate poreux de magnésium et/ou de calcium. Ce document ne mentionne pas l'application de ces compositions pour la coloration artificielle de la peau.

On connaît dans la demande de brevet WO 01/43711 des compositions pour le soin de la peau contenant l'association d'un extrait du Monascus et d'un dérivé de l'acide ascorbique (ascorbyl palmitate, sodium ascorbyl phosphate) ou d'un dérivé du rétinol. (rétinol ou l'un de ses esters). Ces compositions sont utilisées pour leur activité régulatrice du sébum de la peau, leur activité stimulante de la synthèse collagène par les fibroblastes de la peau, leurs propriétés phytoestrogèniques. Ce document ne mentionne pas l'application de ces compositions pour la coloration artificielle de la peau.

A la suite d'importantes recherches menées dans le domaine de la coloration artificielle de la peau, la Demanderesse a maintenant découvert que l'utilisation à titre d'agent de coloration de la peau de pigments susceptibles d'être obtenus par extraction par un solvant organique ou hydro-organique d'un milieu de culture de micromycètes du type monascus, permettait de conférer immédiatement après l'application sur la peau du produit une coloration durable et non-couvrante, proche du bronzage naturel.

La présente invention a donc pour objet une composition cosmétique et/ou dermatologique, produisant sur la peau une coloration durable et non couvrante, proche du bronzage naturel, caractérisée par le fait qu'elle comprend, dans un support cosmétiquement acceptable au moins un pigment susceptible d'être obtenu par extraction par un solvant organique ou hydro-organique d'un milieu de culture de micromycètes du type monascus.

La présente invention a encore pour objet l'utilisation d'au moins un pigment un pigment susceptible d'être obtenu par extraction par un solvant organique ou hydro-organique d'un milieu de culture de micromycètes du type monascus, dans une composition cosmétique dans le but de conférer à la peau une coloration durable et non-couvrante proche du bronzage naturel.

La présente invention a également pour objet un procédé de coloration de la peau durable, non-couvrante et proche du bronzage naturel, caractérisé en ce qu'il consiste à appliquer sur celle-ci une quantité efficace d'au moins un pigment susceptible d'être obtenu par extraction par un solvant organique ou hydro-organique d'un milieu de culture de micromycètes du type monascus dans une composition cosmétique le contenant.

Les compositions et les utilisations conformes à l'invention permettent d'obtenir une coloration artificielle proche du bronzage naturel en un laps de temps court. Ainsi, on obtient une coloration immédiate qui permet une visualisation de l'application et par conséquent une meilleure homogénéité dans l'étalement de la composition sur la peau et donc de la coloration qui en résulte. De plus, la coloration artificielle obtenue sur la peau selon l'invention est extrêmement proche du bronzage naturel.

Au sens de la présente invention, on entendra, par « composition produisant sur la peau une coloration durable et non-couvrante, proche du bronzage naturel», une formulation ayant une affinité particulière pour la peau lui permettant de conférer à cette dernière

- une coloration qui ne s'élimine ni à l'eau ni à l'aide d'un solvant et qui résiste à la fois au frottement et au lavage par une solution contenant des tensioactifs ;
- une coloration n'ayant pas tendance à opacifier la peau.

Une telle coloration durable et non couvrante se distingue donc de la coloration superficielle, momentanée et opacifiante apportée par exemple par un produit de maquillage.

La coloration proche du bronzage naturel de la peau pourra être caractérisée par un test in vitro sur un support du type Vitro-Skin®. Vitro-Skin® est un support développé pour la recherche qui reproduit les propriétés de surface de la peau humaine. Il contient à la fois des composants protéiniques et lipidiques optimisés et est conçu avec une topographie, un pH, une tension critique de surface et une force ionique similaire à ceux de la peau humaine. Vitro-Skin® est fournie par l'IMS (IMS Testing Group, USA).

D'autres caractéristiques, aspects et avantages de la présente invention apparaîtront à la lecture de la description détaillée qui va suivre.

Les compositions conformes à la présente invention de façon générale contiennent le ou les pigments susceptibles d'être obtenus par extraction par un solvant organique ou hydro-organique d'un milieu de culture de micromycètes du type monascus dans une quantité efficace permettant d'obtenir au bout de 15 minutes après application sur un support du type Vitro-Skin® à raison de 2mg/cm$^2$ un assombrissement caractérisé dans le système de mesure colorimétrique (L*, a*, b*) par un $\Delta L^*$ allant de -0,5 à -20. De façon préférentielle, $\Delta L^*$ variera de - 0,5 à - 15.

Les compositions conformes à la présente invention apportent au bout de 15 minutes après application sur un support du type Vitro-Skin® à raison de 2mg/cm$^2$ une coloration définie dans le système de mesure colorimétrique (L*, a*, b*), par un rapport $\Delta a^*/\Delta b^*$ allant de 0,5 à 6 et encore plus particulièrement allant de 0, 8 à 6.

Dans le système de mesure colorimétrique (L*, a*, b*) :

L* représente la luminance ou clarté, a* représente l'axe rouge-vert (-a*= vert, +a*= rouge) et b* représente l'axe jaune-bleu (-b*=bleu, +b*=jaune). Ainsi, a* et b* expriment la nuance de la peau.

$\Delta L^*$ traduit l'assombrissement de la couleur : plus le $\Delta L^*$ est négatif, plus la couleur s'est assombrie avec :

$$\Delta L^* = L^* \text{ support non coloré} - L^* \text{ support colorée}$$

Le rapport $\Delta a^*/\Delta b^*$ traduit l'équilibre rouge/jaune et donc la nuance avec :

$\Delta a^*$ = a* support non coloré - a* support coloré

$\Delta b^*$ = b* support non coloré - b* support coloré

Parmi les pigments issus du genre monascus, utilisables selon la présente invention, on peut citer :

(i) les pigments jaunes répondant à l'une des formules (Ia), (Ib) ou (Ic) suivante :

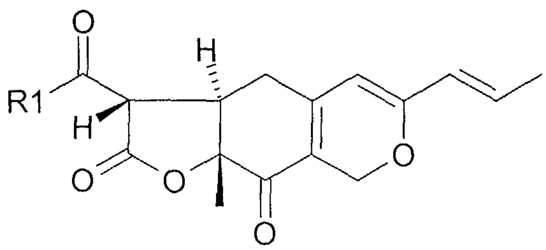

(Ia)

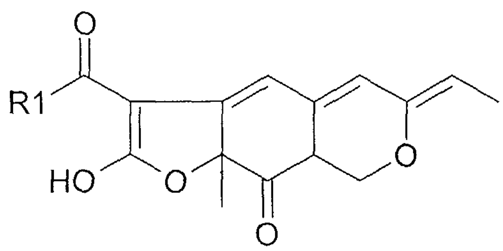

(Ib)

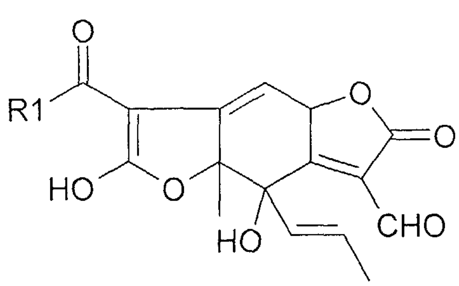

(Ic)

dans chacune desquelles R1 représente un atome d'hydrogène ou un radical alkyle linéaire ou ramifié en $C_1$-$C_{10}$. Préférentiellement, on utilisera :

- les pigments de formule (Ia) où R1 est un radical alkyle linéaire en $C_5$ (monascine) ou un radical alkyle linéaire en $C_7$ (ankaflavine).
- les pigments de formule (Ib) et de formule (Ic) où R1 est un radical alkyle linéaire en $C_5$ ou $C_7$ comme ceux décrits et préparés dans l'article « Secondary métabolites of the fungus Monascus : a review ; P. Juzlova, L.

Martinkova and V. Kren ; Journal of industrial microbiology-1996-16-163-170.»

(ii) les pigments oranges de formule (II) suivante :

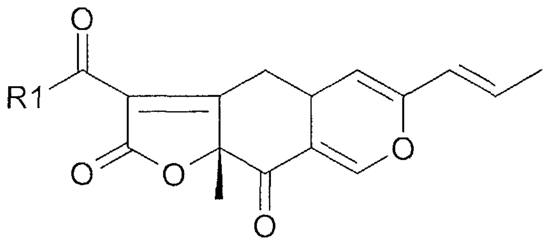

(II)

dans laquelle R1 représente un atome d'hydrogène ou un radical alkyle en $C_1$-$C_{10}$.

Préférentiellement, on utilisera les pigments de formule (I) où R1 est un radical alkyle linéaire en C5 (rubropunctatine) ou un radical alkyle linéaire en C7 (monascorubrine).

(iii) les pigments rouges de formule (III) suivante :

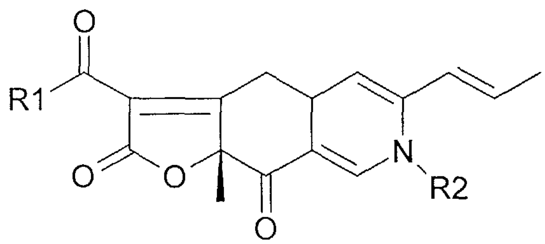

(III)

dans laquelle R1 représente un atome d'hydrogène ou un radical alkyle linéaire ou ramifié en $C_1$-$C_{10}$ ; R2 représente un atome d'hydrogène ou un radical alkyle linéaire ou ramifié en $C_1$-$C_{30}$ ledit radical alkyle pouvant être substitué par une ou plusieurs fonctions acide carboxylique, acide sulfonique, amine et/ou par un ou plusieurs cycles contenant de 4 à 10 atomes de carbone .

Préférentiellement, on utilisera les pigments de formule (III) où R1 est un radical alkyle linéaire en $C_5$ ou un radical alkyle en $C_7$.

Parmi ces pigments rouges, on peut citer ceux pour lesquels R2 est un reste d'acide aminé comme par exemple les pigments du type N-glutaryl monascorubramine, N acetyl monascorubramine et N acetyl rubropunctamine.

Parmi ces pigments rouges on peut également citer ceux pour lesquels R2 est un hydrogène comme par exemple, la rubropunctamine et la monascorubramine.

Les pigments conformes à l'invention sont obtenus par extraction par un solvant organique ou hydro-organique d'un milieu de culture de champignons filamenteux du genre Monascus produit par fermentation. Deux types de procédé de fermentation peuvent être utilisés : la fermentation en milieu liquide ou en milieu solide selon les procédés tels que décrits dans les brevets US 4,145,254 ; JP10-194928, JP4-23880 (faisant partie intégrante de la description). L'extraction par un solvant organique ou hydroorganique de ces milieux permet d'obtenir des pigments dépourvus de toutes substances protéiques telles que les protéines, les peptides et les enzymes. Le solvant d'extraction est constitué d'un ou plusieurs solvants organiques et peut contenir éventuellement de l'eau. Les solvants organiques utilisés peuvent être des alcools comme l'éthanol, le méthanol, l'alcool propylique normal primaire, l'alcool isopropylique, l'alcool butylique normal primaire, le propylène glycol et le glycérol par exemple. Les solvants organiques peuvent être également représentés par l'éther diéthylique, l'acétone, l'éthylméthylcétone, l'acétate d'éthyle par exemple. Les solvants organiques utilisés peuvent également être des fluides supercritiques ou des solvants fluorées comme le dodécafluoropentane, le tétradécafluorohexane, la N-méthyl morpholyne perfluoré et le méthoxy nonafluorobutane par exemple.

Les souches productrices de tels pigments peuvent être choisies parmi celles du type *Monascus purpureus, Monascus ruber, Monascus major, Monascus rubiginosus, Monascus species, Monascus anka et* plus particulièrement celles figurant dans la liste suivante :

*Monascus purpureus*

CCM 8152
CCM 8152/10
ATCC 16365
ATCC 6405
ATCC 16427
NBIMC 23255 (94-25)
IFO 4513
OUT 2013
OUT 2014

*Monascus ruber*

CCM 8111
CCM 8112
ATCC96218
DSM62748

*Monascus major*

ATCC 16362

*Monascus rubiginosus*

ATCC 16367

Monascus species

ATCC 16435

*Monascus anka*

ATCC 16360
IFO 6540
IAM 8002
IFO 4473

Les pigments selon l'invention sont plus particulièrement choisis parmi les pigments rouges de formule (III) tels que définis précédemment et encore plus particulièrement un extrait hydroalcoolique de Monascus anka atomisé sur maltodextrine (4/96)) tel que le produit commercial Monacolor MS-60 de ICHIMARU PHARCOS.

La concentration en pigment dérivé de monascus tel que décrit selon la présente invention, varie de préférence de 0,0001 à 10%, et encore plus préférentiellement de 0,001 à 5% en poids, par rapport au poids total de la composition.

Selon une forme particulière de l'invention, dans les compositions de l'invention ledit pigment dérivé de monascus sera différent d'un extrait issu de la souche mutante R300-30 du Monascus anka (FERM n° 11135) tel que décrit dans la demande de brevet JP-4-23880. Selon une autre forme de l'invention, le pigment de Monascus ne sera pas lié à une poudre de fibroïne de soie régénérée comme le préconisent les demandes de brevet P 60-149512 et JP10-194928 ou à une poudre de silicate d'aluminate poreux de magnésium et/ou de calcium comme le préconise la demande de brevet JP 57-145159. Selon une autre forme de l'invention, les compositions de l'invention ne contiendront pas de dérivé de l'acide ascorbique ni de dérivé du rétinol telles que les formulations décrites dans la demande de brevet WO 01/43711.

Les compositions de l'invention peuvent contenir en plus au moins un autobronzant mono ou polycarbonylé.

Les agents autobronzants mono ou polycarbonylés sont choisis par exemple parmi l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrits dans la demande de brevet FR 2466492 et WO 9735842, la dihydroxyacétone (DHA), les dérivés de 4,4-dihydroxypyrazolin-5-ones telles que décrits dans la demande de brevet EP903342, ces agents autobronzants pouvant être associés ou non à des colorants directs ou des dérivés indoliques.

Dans un mode de réalisation préféré de l'invention on utilisera plus particulièrement la dihydroxyacétone (DHA).

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition.

Les compositions de l'invention peuvent également contenir en plus au moins un agent filtrant les radiations UV, organique ou minéral.

Les filtres UV organiques conformes à l'invention peuvent être hydrosolubles, liposolubles ou insolubles dans les solvants usuels cosmétiques. Ils sont choisis notamment parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone notamment ceux décrits dans les demandes EP-A-1046391 et DE10012408 ; les dérivés de benzalmalonate, les dérivés de β,β'-diphénylacrylate, les dérivés de benzotriazole, les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis- (hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665 ; les dimères dérivés d'α-alkylstyrène tels que ceux décrits dans la demande de brevet DE19855649 ; les dérivés de 4,4-diarylbutadiène tels que ceux décrits dans les demandes de brevet EP0967200, DE19755649, EP1333981.

Comme exemples de filtres organiques, on peut citer désignés ci-dessus sous leur nom INCI :

Dérivés de l'acide para-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

Dérivés salicyliques :

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

Dérivés du dibenzoylméthane :

- Butyl Methoxydibenzoylmethane vendu notamment sous le nom commercial « PARSOL 1789 » par HOFFMANN LA ROCHE,
- Isopropyl Dibenzoylmethane,

Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,

- - Diisopropyl Methylcinnamate,
- Glyceryl Ethylhexanoate Dimethoxycinnamate

Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12
- le 2-(4-diéthylamino-2-hydroxybenzoyl)-benzoate de n-hexyle.

Dérivés du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

Dérivés du phenyl benzimidazole :

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial commercial « NEO HELIOPAN AP » par HAARMANN et REIMER,

Dérivés de la triazine :

- Anisotriazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

Dérivés du phenyl benzotriazole :

- Drometrizole Trisiloxane vendu sous le nom « SILATRIZOLE » par RHODIA CHIMIE ,

- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés anthraniliques :

- Menthyl anthranilate vendu sous le nom commercial commercial « NEO HELIOPAN MA » par HAARMANN et REIMER,

Dérivés d'imidazolines :

- Ethylhexyl Dimethoxybenzylidene Dioxoimidazoline Propionate,

Dérivés du benzalmalonate :

- Polyorganosiloxane à fonctions benzalmalonate vendu sous la dénomination commerciale « PARSOL SLX » par HOFFMANN LA ROCHE

Dérivés de 4,4-diarylbutadiène :

- 1,1-dicarboxy (2,2'-diméthyl-propyl)-4,4-diphénylbutadiène et leurs mélanges.

Les filtres UV organiques plus particulièrement préférés sont choisis parmi les composés suivants :

- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Disodium Phenyl Dibenzimidazole Tetra-sulfonate
- Anisotriazine,
- Ethylhexyl triazone,
- Diethylhexyl Butamido Triazone,
- la 2,4,6-tris-(4'-amino benzalmalonate de diisobutyle)-s- triazine.
- Methylene bis-Benzotriazolyl Tetramethylbutylphenol,
- le 2-(4-diéthylamino-2-hydroxybenzoyl)-benzoate de n-hexyle,
- 1,1-dicarboxy (2,2'-diméthyl-propyl)-4,4-diphénylbutadiène
- Drometrizole Trisiloxane, et leurs mélanges.

Les agents filtrant minéraux sont généralement des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de

brevets EP-A-0518772 et EP-A-0518773.

Les agents filtrants les radiations conformes à l'invention sont généralement présents dans les compositions selon l'invention dans des proportions allant de 0,1 à 20% en poids par rapport au poids total de la composition, et de préférence allant de 0,2 à 15% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les $\alpha$-hydroxyacides, les agents répulsifs contre les insectes, les agents anti-mousse, les agents hydratants, les vitamines, les parfums, les conservateurs, les tensioactifs, les charges, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en $C_{12}$-$C_{15}$ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus et/ou leurs quantités de manière telle que les propriétés avantageuses attachées intrinsèquement à l'utilisation des pigments conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. **13**, 238 (1965), FR 2 315 991 et FR 2 416 008).

Des exemples concrets, mais nullement limitatifs, illustrant l'invention, vont maintenant être donnés.

**EXEMPLE 1 :**

La Demanderesse a réalisé les compositions suivantes (les quantités sont exprimées en pourcentage de poids par rapport au poids total de la composition) :

Composition $A_1$ (hors invention):

- Dihydroxyacétone (DHA) 1,00 g
- Ethanol absolu 49, 50g
- Propylène glycol 24,75 g
- Eau déminéralisée 24,75g

Composition $B_1$(invention)

- Pigment monascus anka (Monacolor MS-60 de ICHIMARU PHARCOS) 1,00g
- Ethanol absolu 49, 50g
- Propylène glycol 24,75 g
- Eau déminéralisée 24,75g

**Protocole d'évaluation :**

Préparation de VitroSkin ®

Pour utiliser Vitro Skin dans le test in vitro de caractérisation de la couleur il faut préalablement l'hydrater.

Réalisation du liquide d'hydratation

Le bas d'un dessiccateur est rempli avec un mélange eau/glycérine dans les proportions suivantes (70/30).

Hydratation de VitroSkin ®

Des carrés de VitroSkin ® préalablement découpés sont déposés sur un support dans le dessicateur clos contenant le liquide d'hydratation. Une période de 16 heures d'hydratation est nécessaire pour utiliser le Vitroskin ®.
Les compositions $A_1$ et $B_1$ ont ensuite été appliquées sur les carrés de VitroSkin ® prélevés dans le dessiccateur à raison de 2 $mg/cm^2$ sur une zone de 2,5 x 2,5 $cm^2$.
Les cinq séries de mesures colorimétriques suivantes ont été effectuées à l'aide d'un colorimètre Minolta CM-508d :

- 1°) avant application de la composition,
- 2°) 15 minutes après l'application,

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.
Pour l'évaluation de l'intensité de la coloration, on s'intéresse à ΔL* qui traduit l'assombrissement de la couleur : plus ΔL* est négatif, plus la couleur s'est assombrie avec :

$$\Delta L^* = L^* \text{ Vitro Skin ® non coloré} - L^* \text{ Vitro Skin ® coloré}$$

Pour la nuance de la coloration obtenue, on s'intéresse au rapport Δa*/Δb* qui traduit l'équilibre rouge/jaune et donc la nuance avec :

$$\Delta a^* = a^* \text{ Vitro Skin ® non coloré} - a^* \text{ Vitro Skin ® coloré}$$

$$\Delta b^* = b^* \text{ Vitro Skin ® non coloré} - b^* \text{ Vitro Skin ® coloré}$$

Les résultats obtenus sont rassemblés dans le tableau (I) suivant :

Tableau (I) :

| | **Composition $A_1$ (comparative)** | **Composition $B_1$ (invention)** |
|---|---|---|
| **ΔL*** | -0,99 | -8.4 |
| **Δa*/Δb*** | 0,06 | 5.8 |

On constate ainsi que 15 minutes après l'application, la composition $A_1$, qui contient à titre d'agent de coloration de la peau, la DHA, n'a conféré qu'une très faible coloration à Vitro Skin ®, puisque la DHA n'a pas encore eu le temps d'agir ( ΔL* = -0.99). En revanche, la composition $B_1$ selon l'invention permet d'obtenir immédiatement sur Vitro Skin ® une coloration de bonne intensité (ΔL*= -8.4).

**EXEMPLE 2 :**

La Demanderesse a réalisé les compositions suivantes (les quantités sont exprimées en pourcentage de poids par rapport au poids total de la composition) :

Composition $A_2$ (hors invention):

- Dihydroxyacétone (DHA) 1.0 g
- Ethanol absolu 49, 50g
- Propylène glycol 24,75 g
- Eau déminéralisée 24,75g

Composition $B_2$ (invention)

- Pigment rouge monascus anka 1,00g (Monacolor MS-60 de ICHIMARU PHARCOS)
- Dihydroxyacétone (DHA) 1,00g
- Ethanol absolu 49,2g
- Propylène glycol 24,4 g
- Eau déminéralisée 24,4g

On réalise les mêmes types de tests que ceux décrits dans l'exemple 1.
Les résultats obtenus sont rassemblés dans le tableau (II) suivant :

Tableau (II) :

| | **Composition $A_2$ (comparative)** | **Composition $B_2$ (invention)** |
|---|---|---|
| **ΔL*** | -0,99 | -7.8 |
| **Δa*/Δb*** | -0,06 | 4.2 |

On constate ainsi que 15 minutes après l'application, la composition $A_2$, qui contient à titre d'agent de coloration de la peau, la DHA, n'a conféré qu'une très faible coloration sur Vitro Skin ®, puisque la DHA n'a pas encore eu le temps d'agir ( ΔL* = -0.99). En revanche, la composition $B_2$ selon l'invention permet d'obtenir immédiatement sur Vitro Skin ® une coloration de bonne intensité (ΔL*= -7.8).

**EXEMPLE 3 :**

La Demanderesse a réalisé les compositions suivantes (les quantités sont exprimées en pourcentage de poids par rapport au poids total de la composition) :
Composition $A_3$ (hors invention):

- Dihydroxyacétone (DHA) 1,00 g
- Ethanol absolu 49, 50g
- Propylène glycol 24,75 g
- Eau déminéralisée 24,75g

Composition $B_3$ (invention)

- Pigment monascus 1,00g (Monascus Red de l'institut botanique de Kunming)
- Terephtalylidene dicamphor sulfonic acid 0,5 g
- Ethanol absolu 49,3g
- Propylène glycol 24,6 g
- Eau déminéralisée 24,6g

On réalise les mêmes types de tests que ceux décrits dans l'exemple 1.

Les résultats obtenus sont rassemblés dans le tableau (III) suivant :

Tableau (III) :

| | **Composition $A_3$ (comparative)** | **Composition $B_3$ (invention)** |
|---|---|---|
| **ΔL*** | -0,99 | -10,64 |
| **Δa*/Δb*** | 0,06 | 4,6 |

On constate ainsi que 15 minutes après l'application, la composition $A_3$, qui contient à titre d'agent de coloration de la peau, la DHA, n'a conféré qu'une très faible coloration à VitroSkin ®, puisque la DHA n'a pas encore eu le temps d'agir ( ΔL* = -0.99). En revanche, la composition $B_3$ selon l'invention permet d'obtenir immédiatement sur VitroSkin ®une coloration de bonne intensité (ΔL*= -10,64).

**Revendications**

1. Composition cosmétique et/ou dermatologique, produisant sur la peau une coloration durable et non-couvrante, proche du bronzage naturel, **caractérisée par le fait qu'**elle comprend, dans un support cosmétiquement acceptable, au moins un pigment susceptible d'être obtenu par extraction par un solvant organique ou hydro-organique d'un milieu de culture de micromycètes du type monascus.

2. Composition selon la revendication 1, **caractérisée par le fait qu'**elle comprend ledit pigment dans une quantité efficace permettant d'obtenir au bout de 15 minutes après application sur un support du type Vitroskin à raison de $2mg/cm^2$ un assombrissement **caractérisé** dans le système de mesure colorimétrique L* a* b* par un ΔL* allant de - 0,5 à -20.

3. Composition selon la revendication 2, où le ΔL* varie de - 0,5 à -15 .

4. Composition selon la revendication 2 ou 3, selon laquelle ledit pigment est présent dans une quantité efficace permettant d'obtenir au bout de 30 minutes après application sur un support du type Vitroskin à raison de $2mg/cm^2$ une coloration définie dans le système colorimétrique L* a* b* par un rapport Δa*/Δb* allant de 0,5 à 6.

5. Composition selon la revendication 4, où le rapport Δa*/Δb* varie de 0,8 à 6.

6. Composition selon l'une quelconque des revendications 1 à 5, où les pigments issus des mycromycètes du genre monascus sont choisis parmi les pigments jaunes répondant à l'une des formules (Ia), (Ib) ou (Ic) suivante :

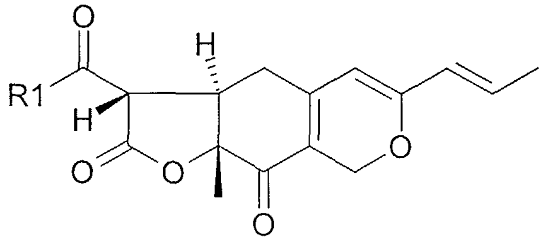

(Ia)

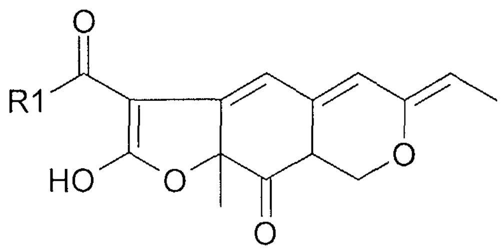

(Ib)

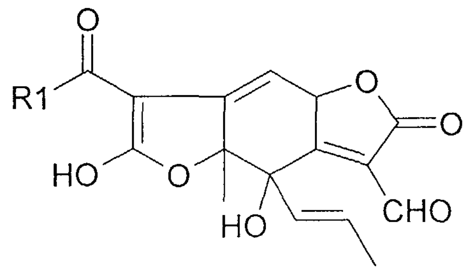

(Ic)

dans chacune desquelles R1 représente un atome d'hydrogène ou un radical alkyle linéaire ou ramifié en $C_1$-$C_{10}$.

**7.** Composition selon la revendication 6, où les pigments issus des mycromycètes du genre monascus sont choisis parmi :

- les pigments de formule (Ia) où R1 est un radical alkyle linéaire en $C_5$ (monascine) ou un radical alkyle linéaire en $C_7$ (ankaflavine) ;
- les pigments de formule (Ib) où R1 est un radical alkyle linéaire en $C_5$ ou un radical alkyle linéaire en $C_7$ ;
- les pigments de formule (Ic) où R1 est un radical alkyle linéaire en $C_5$ ou un radical alkyle linéaire en $C_7$.

**8.** Composition selon l'une quelconque des revendications 1 à 5, où les pigments issus des mycromycètes du genre monascus sont choisis parmi les pigments oranges de formule (II) suivante :

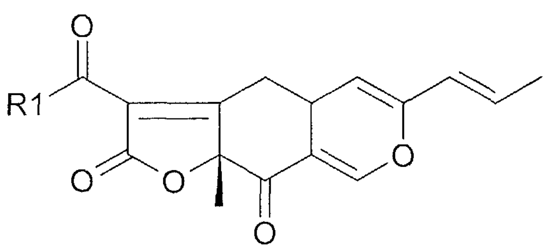

(II)

dans laquelle R1 représente un atome d'hydrogène ou un radical alkyle en $C_1$-$C_{10}$.

**9.** Composition selon la revendication 8, où les pigments oranges de formule (II) sont choisis parmi ceux pour lesquels R1 est un radical alkyle linéaire en $C_5$ (rubropunctatine) ou un radical alkyle linéaire en $C_7$ (monascorubrine).

**10.** Composition selon l'une quelconque des revendications 1 à 5, où les pigments issus des mycromycètes du genre monascus sont choisis parmi les pigments rouges de formule (III) suivante :

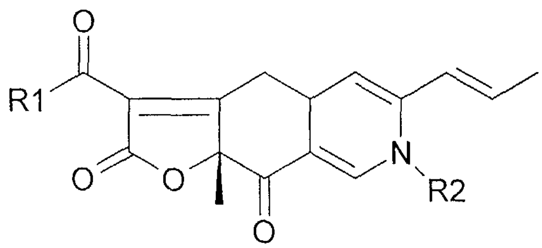

(III)

dans laquelle R1 représente un atome d'hydrogène ou un radical alkyle linéaire ou ramifié en $C_1$-$C_{10}$ ; R2 représente un atome d'hydrogène ou un radical alkyle linéaire ou ramifié en $C_1$-$C_{30}$ ; ledit radical alkyle pouvant être substitué par une ou plusieurs fonctions acide carboxylique, acide sulfonique, amine et/ou par un ou plusieurs cycles contenant de 4 à 10 atomes de carbone.

**11.** Composition selon la revendication 10, où les pigments rouges de formule (III) sont choisis parmi ceux pour lesquels R1 est un radical alkyle linéaire en $C_5$ ou un radical alkyle linéaire en $C_7$.

**12.** Composition selon la revendication 11, où les pigments rouges de formule (III) sont choisis parmi ceux pour lesquels R2 est un reste dérivé d'acide aminé.

**13.** Composition selon la revendication 12, où les pigments rouges de formule (III) sont choisis parmi ceux du type N-glutaryl monascorubramine, N acetyl monascorubramine, N acetyl rubropunctamine.

**14.** Composition selon la revendication 11, où les pigments rouge de la formule (III) sont choisis la rubropunctamine ou la monascorubramine.

**15.** Composition selon l'une quelconque des revendications 1 à 14, où les pigments issus des mycromycètes du genre monascus sont obtenus par fermentation par culture d'une souche choisie parmi celles du type *Monascus purpurea, Monascus* ruber, *Monascus major, Monascus rubiginosus, Monascus species, Monascus anka.*

**16.** Composition selon l'une quelconque des revendications 1 à 15, où le pigment est un extrait hydroalcoolique de Monascus anka atomisé sur maltodextrine (4/96)).

**17.** Composition selon l'une quelconque des revendications 1 à 16, où la concentration en pigment issu de micromycètes du type monascus varie de 0,0001 à 10%, et encore plus préférentiellement de 0,001 à 5% en poids, par rapport au poids total de la composition.

**18.** Composition selon l'une quelconque des revendications 1 à 17, contenant en plus au moins un autobronzant mono ou polycarbonylé.

**19.** Composition selon la revendication 18, où le ou les autobronzants mono ou polycarbonylés sont choisis parmi l'isatine, l'alloxane, la ninhydrine, le glycéraldéhyde, l'aldéhyde mésotartrique, la glutaraldéhyde, l'érthrulose, les dérivés de pyrazoline-4,5-diones, la dihydroxyacétone (DHA), les dérivés de 4,4-dihydroxypyrazolin-5-ones ; ces autobronzants pouvant être associés ou non à des colorants directs ou des dérivés indoliques.

**20.** Composition selon la revendication 19, où l'autobronzant est la dihydroxyacétone (DHA).

**21.** Composition selon l'une quelconque des revendications 18 à 20, où l'autobronzant est présent dans des proportions allant de 0,1 à 10% en poids par rapport au poids total de la composition.

**22.** Composition selon l'une quelconque des revendications 1 à 21, contenant en plus au moins un agent filtrant les radiations UV, organique ou minéral.

**23.** Composition selon la revendications 22, où le ou les agents filtrant les radiations UV organiques sont hydrosolubles, liposolubles ou insolubles dans les solvants usuels cosmétiques.

24. Composition selon la revendication 23, où le ou les agents filtrant les radiations UV organiques sont choisis parmi les anthranilates ; les dérivés cinnamiques ; les dérivés de dibenzoylméthane ; les dérivés salicyliques ; les dérivés du camphre ; les dérivés de triazine ; les dérivés de la benzophénone ; les dérivés de benzalmalonate ; les dérivés de β,β'-diphénylacrylate ; les dérivés de benzotriazole ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène ; les dérivés de 4,4-diarylbutadiène et leurs mélanges.

25. Composition selon la revendication 24, où le ou les agents filtrants les radiations UV organiques sont choisis parmi :

- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Disodium Phenyl Dibenzimidazole Tetra-sulfonate
- Anisotriazine,
- Ethylhexyl triazone,
- Diethylhexyl Butamido Triazone,
- la 2,4,6-tris-(4'-amino benzalmalonate de diisobutyle)-s- triazine.
- Methylene bis-Benzotriazolyl Tetramethylbutylphenol,
- le 2-(4-diéthylamino-2-hydroxybenzoyl)-benzoate de n-hexyle,
- le 1,1-dicarboxy (2,2'-diméthyl-propyl)-4,4-diphénylbutadiène
- Drometrizole Trisiloxane, et leurs mélanges.

26. Composition selon la revendication 22, où le ou les agents filtrant les radiations UV minéraux sont choisis parmi les pigments ou les nanopigments d'oxydes métalliques enrobés ou non.

27. Composition selon la revendication 26, où le ou les agents filtrant les radiations UV minéraux sont choisis parmi les nanopigments d'oxyde de titane, de fer, de zinc, de zirconium ou de cérium, enrobés ou non.

28. Composition selon l'une quelconque des revendications 22 à 27, où le ou les agents filtrant les radiations UV sont présents dans des proportions allant de 0,1 à 15% en poids par rapport au poids total de la composition.

29. Composition selon l'une quelconque des revendications 1 à 28, où ledit pigment issu de micromycètes du type monascus est différent d'un extrait issu de la souche mutante R300-30 du Monascus anka (FERM n° 11135).

30. Composition selon l'une quelconque des revendications 1 à 29, où ledit pigment issu de micromycètes du type monascus n'est pas lié à une poudre de fibroïne de soie régénérée ou à une poudre de silicate d'aluminate poreux de magnésium et/ou de calcium

31. Composition selon l'une quelconque des revendications 1 à 30, ne contenant pas de dérivé de l'acide ascorbique ni de dérivé du rétinol.

32. Procédé de traitement cosmétique de la peau destiné à lui conférer une coloration durable et non-couvrante, proche du bronzage naturel, **caractérisé en ce qu'**il consiste à appliquer sur celle-ci une quantité efficace d'une composition telle que définie à l'une quelconque des revendications 1 à 31.

33. Utilisation d'au moins un pigment susceptible d'être obtenu par extraction par un solvant organique ou hydro-organique d'un milieu de culture de micromycètes du type Monascus tel que défini à l'une quelconque des revendications précédentes dans une composition cosmétique dans le but de conférer à la peau une coloration durable et non-couvrante, proche du bronzage naturel.